# EUROPEAN PATENT SPECIFICATION

(11) **EP 4 015 655 B1**
(45) Date of publication and mention of the grant of the patent: **05.06.2024**
(21) Application number: 20930809.7
(22) Date of filing: 15.10.2020
(51) Int. Cl.: C12Q 1/70, C12Q 1/689, C12Q 1/6851, C12Q 1/06

(54) **COMPOSITION, KIT AND METHOD FOR DETECTING AND CLASSIFYING PATHOGENS CAUSING RESPIRATORY TRACT INFECTIONS, AND APPLICATION**
ZUSAMMENSETZUNG, KIT UND VERFAHREN ZUM NACHWEIS UND ZUR KLASSIFIZIERUNG VON KRANKHEITSERREGERN VERURSACHENDEN PATHOGENEN UND ANWENDUNG
COMPOSITION, KIT ET PROCÉDÉ DE DÉTECTION ET DE CLASSIFICATION D'AGENTS PATHOGÈNES PROVOQUANT DES INFECTIONS DES VOIES RESPIRATOIRES, ET APPLICATION

(30) Priority: 16.04.2020 CN 202010302240
(43) Date of publication of application: 22.06.2022
(73) Proprietor: Sansure Biotech Inc., Changsha, Hunan 410205 (CN)
(72) Inventor: DAI, Lizhong, Changsha High-Tech Industrial Development Zone Hunan 410205 (CN); CHEN, Xiaoliang, Changsha High-Tech Industrial Development Zone Hunan 410205 (CN); REN, Xiaomei, Changsha High-Tech Industrial Development Zone Hunan 410205 (CN); XIANG, Jiangyan, Changsha High-Tech Industrial Development Zone Hunan 410205 (CN); WAN, Wang, Changsha High-Tech Industrial Development Zone Hunan 410205 (CN); LIAO, Shiliu, Changsha High-Tech Industrial Development Zone Hunan 410205 (CN); DENG, Zhongping, Changsha High-Tech Industrial Development Zone Hunan 410205 (CN)
(74) Representative: Novagraaf Technologies
(86) International application number: PCT/CN2020/121011
(87) International publication number: WO 2021/208382

(56) References cited:
- WO-A1-2013/049891
- CN-A- 105 400 907
- CN-A- 109 355 437
- CN-A- 110 982 943
- CN-A- 111 321 251
- CN-B- 107 058 622
- JASPER FUK-WOO CHAN ET AL: "Improved Molecular Diagnosis of COVID-19 by the Novel, Highly Sensitive and Specific COVID-19-RdRp/Hel Real-Time Reverse Transcription-PCR Assay ValidatedIn Vitroand with Clinical Specimens", JOURNAL OF CLINICAL MICROBIOLOGY, vol. 58, no. 5, 4 March 2020 (2020-03-04), pages e00310-20, XP055752336, US ISSN: 0095-1137, DOI: 10.1128/JCM.00310-20
- HSIH WEN-HSIN ET AL: "Featuring COVID-19 cases via screening symptomatic patients with epidemiologic link during flu season in a medical center of central Taiwan", JOURNAL OF MICROBIOLOGY, IMMUNOLOGY AND INFECTION, ELSEVIER, AMSTERDAM, NL, vol. 53, no. 3, 13 March 2020 (2020-03-13) , pages 459-466, XP086170992, ISSN: 1684-1182, DOI: 10.1016/J.JMII.2020.03.008 [retrieved on 2020-03-13]
- CIPITELLI MARCIO DA COSTA ET AL: "SARS-CoV-2 diagnostic diary: from rumors to the first case. Early reports of molecular tests from the military research and diagnostic institute of Rio de Janeiro", MEMORIAS DO INSTITUTO OSWALDO CRUZ, vol. 115, 1 January 2020 (2020-01-01), pages 1-5, XP055913561, BR ISSN: 0074-0276, DOI: 10.1590/0074-02760200200 Retrieved from the Internet: URL:https://www.ncbi.nlm.nih.gov/pmc/artic les/PMC7357545/pdf/1678-8060-mioc-115-e200 200.pdf>
- Chatterjee Pranab, Nagi Nazia, Agarwal Anup, Das Bhabatosh, Banerjee Sayantan, Sarkar Swarup, Gupta Nivedita, Gangakhedkar Ramanr: "The 2019 novel coronavirus disease (COVID-19) pandemic: A review of the current evidence", Indian Journal of Medical Research, IN, vol. 0, no. 0, 1 January 2020 (2020-01-01) , page 0, XP055857645, IN ISSN: 0971-5916, DOI: 10.4103/ijmr.IJMR_519_20

## Description

### TECHNICAL FIELD

The present invention pertains to the field of molecular biological detection, specifically, to the field of detection of pathogens that cause respiratory tract infections. More specifically, the present invention is capable of simultaneous detection and typing of novel coronavirus 2019-nCoV, influenza A virus, influenza B virus, a respiratory adenovirus, a respiratory syncytial virus, and *Mycoplasma pneumoniae.*

### BACKGROUND

Infectious respiratory system diseases are mostly common and frequently-occurring diseases in clinical practice. These diseases have similar clinical symptoms and epidemiological characteristics, and it is difficult to identify and determine the type of pathogens infected from clinical symptoms and by routine laboratory tests. Respiratory pathogens can be transmitted through the air. Pathogens that cause acute respiratory diseases have the characteristics of strong infectivity, rapid transmission, short incubation periods, and acute onset, and may cause a wide range of acute upper and lower respiratory tract diseases and seriously endanger human health. Common respiratory tract pathogens include the following pathogens:
2019 novel coronavirus (2019-nCoV) was discovered in December 2019, and was subsequently named SARS-CoV-2 by the International Committee on Taxonomy of Viruses on February 11th. Coronaviruses are a large family of viruses. Six species were previously known to infect humans, such as those that can cause colds as well as Middle East Respiratory Syndrome (MERS) and Severe Acute Respiratory Syndrome (SARS). The novel coronavirus 2019-nCoV is a new strain of coronavirus that has never been found in humans before. Taxonomically, the virus is a β-type coronavirus in the genus *Coronavirus* of the family *Coronaviridae,* and has an envelope and spikes, and is a linear positive-sense single-stranded RNA ((+)ssRNA) virus according to the genomic cytological characteristic. Since the discovery of the novel coronavirus 2019-nCoV in December 2019, the virus has almost exploded all over the world in just over three months, infecting more than one million people globally and killing more than 50,000 people, raising a great attention in the international community.

Influenza virus, briefly referred to as flu virus, is an RNA virus that causes influenza in humans and animals. Human influenza viruses are divided into three types: types A, B, and C, which are pathogens of influenza (flu). AI, short for avian influenza, is an infectious disease caused by a certain subtype of influenza A virus (also called avian influenza virus). Taxonomically, influenza viruses belong to the genus *Influenzavirus* of the family *Orthomyxoviridae,* and will cause acute upper respiratory tract infections, and spread rapidly through the air, and often cause periodic pandemics around the world, such as the "Spanish Influenza" that killed more than 20 million people worldwide in 1918-1919, the "Asian Influenza" that occurred in 1957, the "Hong Kong Influenza" that occurred in 1968, the "Russian Influenza" occurred in 1977, and H7N9 bird flu occurred in 2013.

Respiratory Syncytial Virus (RSV) is an RNA virus belonging to the genus *Pneumonia* of the family *Paramyxoviridae.* Clinical studies have shown that the RSV is the most common pathogen that causes viral pneumonia, and is more common in infants under three years of age, showing the main symptoms of high fever, rhinitis, pharyngitis, and laryngitis, which later develop into bronchiolitis and pneumonia. A small number of sick children may be complicated by otitis media, pleurisy, and myocarditis. For infected adults and older children, the main manifestation is upper respiratory tract infections.

Adenoviruses (HAdv) belong to the family *Adenoviridae.* According to different immunological, biological, and biochemical characteristics, they are divided into seven subspecies from A to G, a total of 52 serotypes. Different serotypes have different organ affinities and cause corresponding clinical manifestations, and may infect the respiratory tract, gastrointestinal tract, urethra, bladder, eyes, and liver.

*Mycoplasma pneumoniae* (MP) is a common pathogen of community-acquired pneumonia. The infection with MP may cause atypical pneumonias. The pathological changes are mainly interstitial pneumonia, sometimes complicated by bronchial pneumonia. MP is mainly transmitted by droplets, with an incubation period of two to three weeks, and the incidence is highest in adolescents. The clinical symptoms are mild, and most patients only have general respiratory tract symptoms such as headache, sore throat, fever, and cough; a few will have persistent high fever and severe cough, show rapid disease progression, and develop into respiratory failure, multiple organ dysfunction, and other critical illnesses in a short period of time.

Infectious respiratory tract diseases have similar clinical symptoms and epidemiological characteristics, and early diagnosis, early treatment, and early isolation are the fundamentals for treatment and prevention. However, it is difficult to identify and determine the type of pathogens infected from clinical symptoms and by routine laboratory tests, and the culture conditions of pathogens are critical, resulting in a low positive rate of culture, and some pathogens even cannot be cultured under current conditions, which pose great difficulty for patients with respiratory tract infections and clinicians.

Therefore, there is an urgent need in the art for a simple, rapid, and objective method for detecting a respiratory pathogen, so as to achieve pathogen determination, early diagnosis, and rational guidance of clinical medication.

### SUMMARY

In view of this, provided in the present invention is a composition capable of detecting and typing pathogens that cause a respiratory tract infection. The composition includes:
a first nucleic acid composition:
   an influenza A virus forward primer as shown in SEQ ID NO: 1, an influenza A virus reverse primer as shown in SEQ ID NO: 2, and an influenza A virus probe as shown in SEQ ID NO: 3;
   an influenza B virus forward primer as shown in SEQ ID NO: 4, an influenza B virus reverse primer as shown in SEQ ID NO: 5, and an influenza B virus probe as shown in SEQ ID NO: 6;
   a respiratory syncytial virus forward primer as shown in SEQ ID NO: 7, a respiratory syncytial virus reverse primer as shown in SEQ ID NO: 8, and a respiratory syncytial virus probe as shown in SEQ ID NO: 9; and
a second nucleic acid composition:
   a respiratory adenovirus forward primer as shown in SEQ ID NO: 10, a respiratory adenovirus reverse primer as shown in SEQ ID NO: 11, and a respiratory adenovirus probe as shown in SEQ ID NO: 12;
   a novel coronavirus 2019-nCoV forward primer as shown in SEQ ID NO: 13, a novel coronavirus 2019-nCoV reverse primer as shown in SEQ ID NO: 14, and a novel coronavirus 2019-nCoV probe as shown in SEQ ID NO: 15;
   a *Mycoplasma pneumoniae* forward primer as shown in SEQ ID NO: 16, a *Mycoplasma pneumoniae* reverse primer as shown in SEQ ID NO: 17, and a *Mycoplasma pneumoniae* probe as shown in SEQ ID NO: 18;
   wherein fluorescent groups of the first nucleic acid composition are different from each other and do not interfere with each other, and fluorescent groups of the second nucleic acid composition are different from each other and do not interfere with each other.

Herein, the description "different from each other and do not interfere with each other" means that the fluorescent groups used by the respective probes in the nucleic acid composition are different, and will not affect the detection of each other, i.e., different channels can be used for detection. For example, FAM, HEX, ROX, and CY5 may be used. The absorbance values of these groups are not close, and different channels can be selected, and therefore, the groups will not interfere with each other.

Further, the composition includes: an internal standard forward primer, internal standard reverse primer, and internal standard probe for monitoring.

In one specific embodiment, the composition further includes: the internal standard forward primer as shown in SEQ ID NO: 19, the internal standard reverse primer as shown in SEQ ID NO: 20, and the internal standard probe as shown in SEQ ID NO: 21.

In the present invention, the fluorescent reporter group may be selected from FAM, HEX, ROX, VIC, CY5, 5-TAMRA, TET, CY3, and JOE, but is not limited thereto.

In one specific embodiment, the fluorescent reporter group of the influenza A virus probe as shown in SEQ ID NO: 3 is FAM; the fluorescent reporter group of the influenza B virus probe as shown in SEQ ID NO: 6 is HEX; and the fluorescent reporter group of the respiratory syncytial virus probe as shown in SEQ ID NO: 9 is CY5.

In one specific embodiment, the fluorescent reporter group of the respiratory adenovirus probe as shown in SEQ ID NO: 12 is FAM; the fluorescent reporter group of the novel coronavirus 2019-nCoV probe as shown in SEQ ID NO: 15 is HEX; and the fluorescent reporter group of the *Mycoplasma pneumoniae* probe as shown in SEQ ID NO: 18 is CY5.

In one specific embodiment, a fluorescent reporter group of the internal standard probe as shown in SEQ ID NO: 21 is ROX.

The primer and probe of the internal standard detect the gene sequence of a human housekeeping gene GAPDH. The GAPDH gene sequence (SEQ ID NO: 22) is:

TCTCCTCTGACTTCAACAGCGACACCCACTCCTCCACCTTTGACGCTGG
GGCTGGCATTGCCCTCAACGACCACTTTGTCAAGCTCATTTCCTGGTATGACAAC
GAATTTGGCTACAGCAACAGGGTGGTGG

Further, the amount of the detection primer in the composition is 25-500 nM; the amount of the detection probe in the composition is 20-500 nM; the amount of the internal standard primer in the composition is 12.5-250 nM; and the amount of the internal standard probe in the composition is 10-250 nM.

In the present invention, the terms "detection primer" and "detection probe" refer to a primer and a probe used for amplifying and detecting a pathogen.

In the present invention, the terms "internal standard primer" and "internal standard probe" refer to a primer and a probe used for amplifying and detecting the internal standard.

In one specific embodiment, the two nucleic acid compositions of the composition of the present invention are each present in separate packages.

Further, the components of each nucleic acid composition of the composition of the present invention are present in a mixed form.

In a second aspect, provided in the present invention is a use of the above composition of the present invention in the preparation of a kit for detecting and typing pathogens that cause a respiratory tract infection.

The pathogens that cause the respiratory tract infection include: a novel coronavirus 2019-nCoV, an influenza A virus, an influenza B virus, a respiratory adenovirus, a respiratory syncytial virus, and *Mycoplasma pneumoniae.*

In a third aspect, provided in the present invention is a kit for detecting and typing pathogens that cause a respiratory tract infection, the kit including the above composition of the present invention.

Further, the kit further includes at least one of a nucleic acid release reagent, a dNTP, a reverse transcriptase, a DNA polymerase, a PCR buffer, and Mg²⁺.

A common PCR buffer is composed of a buffer system such as Tris-HCl, MgCl₂, KCl, and Triton X-100. Generally, the total volume in a single PCR reaction tube is 20-200 µL.

Further, the amount of the detection primer in the composition is 25-500 nM; the amount of the detection probe in the composition is 20-500 nM; the amount of the internal standard primer in the composition is 12.5-250 nM; the amount of the internal standard probe in the composition is 10-250 nM; and the amount of the dNTP is 0.2-0.3 mM.

Further, the concentration of the reverse transcriptase is 5 U/µL to 15 U/µL, and for example, the reverse transcriptase may be a murine leukemia reverse transcriptase (MMLV) or a Tth enzyme. The concentration of the DNA polymerase is 5 U/µL to 15 U/µL, and for example, the DNA polymerase may be a Taq enzyme.

In one specific embodiment, the PCR system of the composition of the present invention is as follows:

| Component | Volume/concentration in each reaction |
|---|---|
| 5 x PCR buffer* | 10 µl |
| dNTPs (100 mM) | 1.5 µl |
| Tth enzyme (5 U/µL) | 1.0 µl |
| H-Taq enzyme (5 U/µL) | 1.0 µl |
| Mn(OAc)₂ (150 mM) | 1.0 µl |
| Detection primer | 0.5 µM |
| Detection probe | 0.25 µM |
| Internal standard primer | 0.25 µM |
| Internal standard probe | 0.1 µM |
| Sterilized purified water | Make up to 45 µl |

Further, the kit contains a positive control and a negative control, and the negative and positive controls and the testing sample need to be processed simultaneously. The positive control is formed by mixing an artificially synthesized lentiviral particle containing specific nucleic acid sequences of a 2019 novel coronavirus, an influenza A virus, an influenza B virus, and a respiratory syncytial virus with an artificially synthesized plasmid containing specific nucleic acid sequences of an adenovirus and *Mycoplasma pneumoniae,* to simulate an actual clinical sample. The negative control consists of an artificially synthesized lentiviral particle containing a target fragment of a GAPDH housekeeping gene (SEQ ID NO: 22), to completely simulate a throat swab sample of a normal person.

In a fourth aspect, a method for detecting and typing pathogens that cause a respiratory tract infection is provided, the method including the steps of:
1) releasing a nucleic acid of a testing sample;
2) performing, by using the above composition of the present invention or the above kit of the present invention, a fluorescent quantitative PCR on the nucleic acid obtained in step 1); and
3) obtaining and analyzing the results.

In the present invention, the testing sample may be a throat swab, sputum, a bronchoalveolar lavage fluid, blood, etc., but is not limited thereto.

Further, reaction conditions of the fluorescent quantitative PCR are as follows:
pre-denaturation and enzyme activation at a temperature of 95°C for 1-10 minutes for 1 cycle; reverse transcription at a temperature of 60°C for 25-35 minutes for 1 cycle; cDNA pre-denaturation at a temperature of 95°C for 1-10 minutes for 1 cycle; denaturation at a temperature of 95°C for 10-20 seconds; annealing at a temperature of 60°C for 20-40 seconds for 40-50 cycles.

In one specific embodiment, a method for detecting and typing pathogens that cause a respiratory tract infection for a non-diagnostic purpose is provided, the method including the steps of:
1) releasing a nucleic acid of a testing sample;
2) performing, by using the above composition of the present invention or the above kit of the present invention, a fluorescent quantitative PCR on the nucleic acid obtained in step 1); and
3) obtaining and analyzing the results.

Using the composition of the present invention can simultaneously detect and type the six pathogens that cause respiratory tract infections, so as to identify some patients having fever caused by common seasonal influenza, reduce the waste of medical resources, and reduce the psychological burden on patients and society.

Furthermore, the present invention can quickly diagnose the novel coronavirus 2019-nCoV, provide molecular evidence for early diagnosis, early treatment, and early isolation of the novel coronavirus, and allow adequate preparations for prevention and control of the disease, making it possible to control the source of infection of the highly contagious and hazardous novel coronavirus 2019-nCoV in a timely manner and stop virus pandemics and outbreaks.

The composition of the present invention in combination with a fluorescent probe method enables the use of two tubes in one test simultaneously, achieving low costs and high throughput. The present invention enables information of four targets to be given by one tube in a single test, and the operations are simple and convenient, and a result reading process can be completed according to a CT value. The whole detection process is carried out under single-tube closed conditions, avoiding false positives and environmental contamination caused by crossover between samples.

### BRIEF DESCRIPTION OF THE DRAWINGS

FIG. 1 is a detection diagram of the composition of the present invention for detecting a novel coronavirus in 400 copies/mL;
FIG. 2 is a sensitivity detection diagram of the composition of the present invention for detecting an influenza A virus in 6.5 TCID₅₀/mL;
FIG. 3 is a sensitivity detection diagram of the composition of the present invention for detecting an influenza B virus in 3.0 TCID₅₀/mL;
FIG. 4 is a detection diagram of the composition of the present invention for detecting a respiratory syncytial virus in 500 copies/mL;
FIG. 5 is a detection diagram diagram of the composition of the present invention for detecting a respiratory adenovirus in 400 copies/mL;
FIG. 6 is a detection diagram of the composition of the present invention for detecting *Mycoplasma pneumoniae* in 400 copies/mL;
FIG. 7 is a specificity detection diagram of the composition of the present invention;
FIG. 8 shows an effect of an interfering substance on detection of a novel coronavirus (including a control curve);
FIG. 9 shows an effect of an interfering substance on detection of an influenza A virus (including a control curve);
FIG. 10 shows an effect of an interfering substance on detection of an influenza B virus (including a control curve);
FIG. 11 shows an effect of an interfering substance on detection of a respiratory syncytial virus (including a control curve);
FIG. 12 shows an effect of an interfering substance on detection of an adenovirus (including a control curve);
FIG. 13 shows an effect of an interfering substance on detection of *Mycoplasma pneumoniae* (including a control curve);
FIG. 14 is a detection result diagram, in which A shows the result of the composition of the present invention, and B shows the result when a rhinovirus is used instead of *Mycoplasma pneumoniae* in the present invention;
FIG. 15 is a detection result diagram, in which A shows the result of the composition of the present invention, and B shows the result when a metapneumovirus is used instead of *Mycoplasma pneumoniae* in the present invention;
FIG. 16 is a detection diagram of the composition of the present invention for detecting six influenza A viruses;
FIG. 17 is a detection diagram of the composition of the comparative example of the present invention for detecting six influenza A viruses;
FIG. 18 is a detection diagram of the composition of the present invention for detecting a novel coronavirus 2019-nCoV in three gradients (2000 copies/mL, 500 copies/mL, and 200 copies/mL); and
FIG. 19 is a detection diagram of a comparative example composition of the present invention for detecting a novel coronavirus 2019-nCoV in three gradients (2000 copies/mL, 500 copies/mL, and 200 copies/mL).

### DETAILED DESCRIPTION

The present invention will be described in detail below with reference to specific embodiments and examples, and the advantages and various effects of the present invention will be more clearly presented therefrom. It should be understood by those skilled in the art that these specific embodiments and examples are intended to illustrate the present invention, rather than to limit the present invention.

### Example 1. Primers and probes used in the present invention

The primers and probes used in the present invention are shown in Table 1 below:

**Table 1**

| Name | Base sequence 5'-3' |
|---|---|
| Influenza A virus forward primer SEQ ID NO: 1 | CAGAAGATTTGTCCTTCCAG |
| Influenza A virus reverse primer SEQ ID NO: 2 | GTCATACTCCTCTGCATTGTCTC |
| Influenza A virus probe SEQ ID NO: 3 | TCTTCGAGCTCTCGGACGAAAAGG CAA |
| Influenza B virus forward primer SEQ ID NO: 4 | GCCATCGGATCCTCAATTC |
| Influenza B virus reverse primer SEQ ID NO: 5 | TTCTTCCGTGACCAGTCTAATT |
| Influenza B virus probe SEQ ID NO: 6 | AAGGACATCCAAAGCCAATTCGAG CAG |
| Respiratory syncytial virus forward primer SEQ ID NO: 7 | CACCCTGACAAAATCAAACAATAT |
| Respiratory syncytial virus reverse primer SEQ ID NO: 8 | CATAGGCACCCATATTGTTAGT |
| Respiratory syncytial virus probe SEQ ID NO: 9 | AGACAAGATGGGCAAATATGGA AAC |
| Respiratory adenovirus forward primer SEQ ID NO: 10 | GCAGTGGTCTTACATGCACATC |
| Respiratory adenovirus reverse primer SEQ ID NO: 11 | TATCCTCACGGTCCACAGGG |
| Respiratory adenovirus probe SEQ ID NO: 12 | CCAGGACGCCTCGGAGTACCTGAG C |
| Novel coronavirus 2019-nCoV forward primer SEQ ID NO: 13 | AGCAATCTTTAATCAGTGTGTAAC AT |
| Novel coronavirus 2019-nCoV reverse primer SEQ ID NO: 14 | AAATCACATGGGGATAGCACTACT |
| Novel coronavirus 2019-nCoV probe SEQ ID NO: 15 | AAAGAGCCACCACATTTTCACCGA GG |
| *Mycoplasma pneumoniae* forward primer SEQ ID NO: 16 | CTTTGGTTCGCATGAATCAA |
| *Mycoplasma pneumoniae* reverse primer SEQ ID NO: 17 | TTCCCTACTGCTGCCTCC |
| *Mycoplasma pneumoniae* probe SEQ ID NO: 18 | TCGTTATTTGATGAGGGTGCGCCAT ATC |
| Internal standard forward primer SEQ ID NO: 19 | CTCCTCTGACTTCAACAGCGA |
| Internal standard reverse primer SEQ ID NO: 20 | CCACCACCCTGTTGCTGT |
| Internal standard probe SEQ ID NO: 21 | TTGACGCTGGGGCTGGCATT |

Among them, fluorescent reporter groups of the influenza A virus probe and the respiratory adenovirus probe were FAM; fluorescent reporter groups of the novel coronavirus 2019-nCoV probe and the influenza B virus probe were HEX; fluorescent reporter groups of the respiratory syncytial virus and the *Mycoplasma pneumoniae* probe were CY5; and a fluorescent reporter group of the internal standard was ROX, and the 3'-end of the probe further had a BHQ1 or BHQ2 quencher group.

### Example 2. Method for detecting and typing pathogens that cause a respiratory tract infection

A testing sample of the present invention was a throat swab, sputum, a bronchoalveolar lavage fluid, or blood. A magnetic bead method was used to extract a viral nucleic acid, and the following operations were performed in a sample processing room:

2.1 An appropriate number of 1.5-mL sterilized centrifuge tubes were taken, and labeled as a negative control, a positive control, and a testing sample, respectively. 300 µL of an RNA extraction solution 1 was added to each tube.

2.2 200 µL of the testing sample or the negative control or the positive control was added to each tube. The tube was covered with a cap, and shaken for 10 seconds for through mixing, and subjected to instant centrifugation.

2.3 100 µL of an RNA extraction solution 2-mix was added to each tube (sucked up after through mixing), and the tube was shaken for 10 seconds for through mixing, and left to stand for 10 minutes at room temperature.

2.4 After instant centrifugation, the centrifuge tubes were placed on a separator, and the solution was slowly sucked out after 3 minutes (be careful not to touch a brown substance adhered to the tube wall).

2.5 600 µL of an RNA extraction solution 3 and 200 µL of an RNA extraction solution 4 were added to each tube, and the tube was shaken for 5 seconds for through mixing and subjected to instant centrifugation, and then the centrifuge tube was placed on the separator again.

2.6 After about 3 minutes, the supernatant separated into two layers. A pipette tip was inserted into the bottom of the centrifuge tube, the liquid was slowly sucked up from the bottom completely and discarded. The tube was left to stand for 1 minute and then the residual liquid at the bottom of the tube was completely sucked up and discarded.

2.7 50 µL of a TE buffer (pH 8.0) was added to each tube to perform elution, and then all the eluted brown mixture was transferred as a testing sample to a 0.2-mL PCR reaction tube, and the tube was covered with a cap and transferred to an amplification test zone.

The real-time fluorescent PCR reaction system was configured as follows:

| Component | Volume/concentration in each reaction |
|---|---|
| 5 x PCR buffer | 10 µl |
| dNTPs (100 mM) | 1.5 µl |
| Tth enzyme (5 U/µL) | 1.0 µl |
| H-Taq enzyme (5 U/µL) | 1.0 µl |
| Mn(OAc)₂ (150 mM) | 1.0 µl |
| Detection primer | 0.5 µM |
| Detection probe | 0.25 µM |
| Internal standard primer | 0.25 µM |
| Internal standard probe | 0.1 µM |
| Sterilized purified water | Make up to 45 µl |

The PCR amplification program was set up as follows:

| Step | | Temperature | Time | Number of cycles |
|---|---|---|---|---|
| 1 | Pre-denaturation and enzyme activation | 95°C | 1 minute | 1 |
| 2 | Reverse transcription | 60°C | 30 minutes | 1 |
| 3 | cDNA pre-denaturation | 95°C | 1 minute | 1 |
| 4 | Denaturation | 95 °C | 15 seconds | 45 |
| | Annealing, extension, and fluorescence collection | 60°C | 30 seconds | |
| 5 | Instrument cooling | 25°C | 10 seconds | 1 |

### Result analysis:

1) Target detection signals were FAM, HEX (or VIC), and CY5, and an internal reference detection signal was ROX.
2) Baseline setting: The baseline was generally set to 3-15 cycles, which specifically could be adjusted according to actual situations. The adjustment principle was to select a region where a fluorescence signal is relatively stable before exponential amplification, a starting point (Start) avoiding signal fluctuation in an initial stage of fluorescence collection, and an ending point (End) being less by 1-2 cycles than the Ct of a sample showing earliest exponential amplification. Threshold setting: The setting principle was to make a threshold line just exceed the highest point of a normal negative control.
3) It was first analyzed whether an amplification curve is detected for the internal standard in the ROX channel and Ct ≤ 39, and if so, it indicated that the current test was effective, and subsequent analysis continued to be carried out:
   A) if a typical S-shaped amplification curve was detected in the FAM channel and Ct < 39, it indicated that the influenza A virus and respiratory adenovirus detection results were positive;
   B) if a typical S-type amplification curve was detected in the HEX channel and Ct < 39, it indicated that the novel coronavirus 2019-nCoV and influenza B virus detection results were positive; and
   C) if a typical S-type amplification curve was detected in the CY5 channel and Ct < 39, it indicated that the respiratory syncytial virus and *Mycoplasma pneumoniae* detection results were positive;
4) If a Ct for the internal standard was not detected in the ROX channel or Ct > 39, it indicated that the concentration of the testing sample was excessively low or there was an interfering substance that inhibited the reaction, and the experiment needed to be re-prepared.

### Example 3. Detection results of clinical samples tested by the composition of the present invention

The composition in Table 1 of the present invention was used to test 411 specimens according to the method described in Example 2, the results were one 2019 novel coronavirus cell culture solution, 25 positive cases of influenza A virus, 68 positive cases of influenza B virus, 77 positive cases of respiratory adenovirus, 18 positive cases of respiratory syncytial virus, and 27 positive cases of *Mycoplasma pneumoniae.* The overall results were consistent with those identified by the Center for Disease Control and Prevention. Additionally, the composition of the present invention was compared with a control method (a method using a commercially available kit as a control kit). While the control method had false negatives or false positives, the detection effects of the composition of the present invention were more accurate. The results are shown in Tables 2-7 below.

**Table 2. Influenza A virus (flu A) negative and positive statistical table**

| Experiment | | Control method | | Total |
|---|---|---|---|---|
| | | Positive | Negative | |
| Kit to be assessed | Positive | 25 | 0 | 25 |
| | Negative | 0 | 385 | 385 |
| Total | | 25 | 385 | 410 |

**Table 3. Influenza B virus (flu B) negative and positive statistical table**

| Experiment | | Control method | | Total |
|---|---|---|---|---|
| | | Positive | Negative | |
| Kit to be assessed | Positive | 67 | 1 | 68 |
| | Negative | 1 | 341 | 342 |
| Total | | 68 | 342 | 410 |

**Table 4. Respiratory syncytial virus (RSV) negative and positive statistical table**

| Experiment | | Control method | | Total |
|---|---|---|---|---|
| | | Positive | Negative | |
| Kit to be assessed | Positive | 18 | 2 | 20 |
| | Negative | 0 | 390 | 390 |
| Total | | 18 | 392 | 410 |

**Table 5. Adenovirus (ADV) negative and positive statistical table**

| Experiment | | Control method | | Total |
|---|---|---|---|---|
| | | Positive | Negative | |
| Kit to be assessed | Positive | 76 | 11 | 87 |
| | Negative | 1 | 322 | 323 |
| Total | | 77 | 333 | 410 |

**Table 6. Mycoplasma pneumoniae (MP) negative and positive statistical table**

| Experiment | | Control method | | Total |
|---|---|---|---|---|
| | | Positive | Negative | |
| Kit to be assessed | Positive | 27 | 2 | 29 |
| | Negative | 0 | 381 | 381 |
| Total | | 27 | 383 | 410 |

**Table 7. Overall sample negative and positive statistical table**

| Experiment | | Control method | | Total |
|---|---|---|---|---|
| | | Positive | Negative | |
| Kit to be assessed | Positive | 214 | 8 | 222 |
| | Negative | 3 | 185 | 188 |
| Total | | 217 | 193 | 410 |

### Example 4. Sensitivity of the composition of the present invention

Experiments were carried out with samples of different concentrations to test the sensitivity of the composition of the present invention.

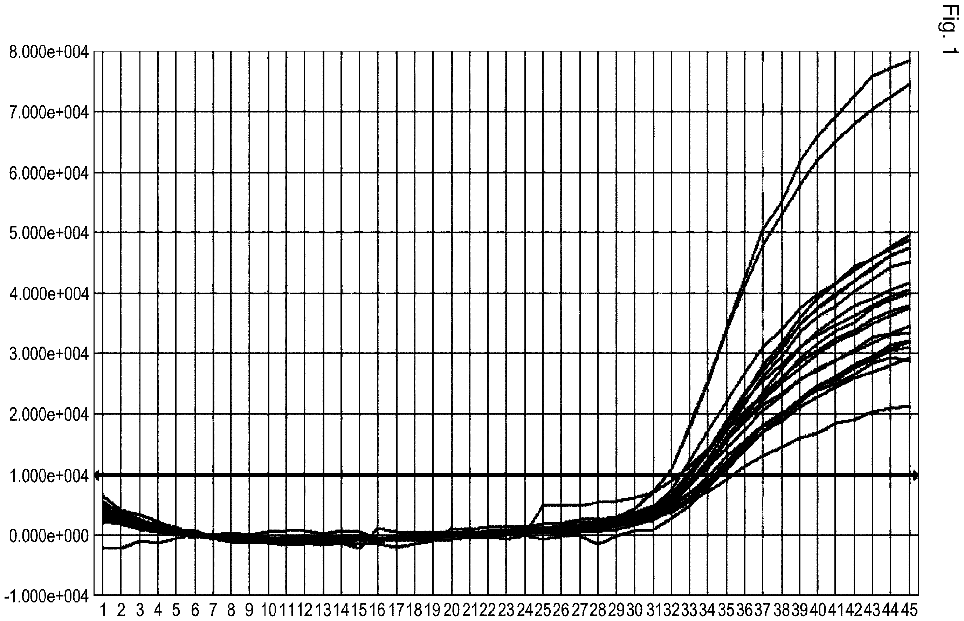

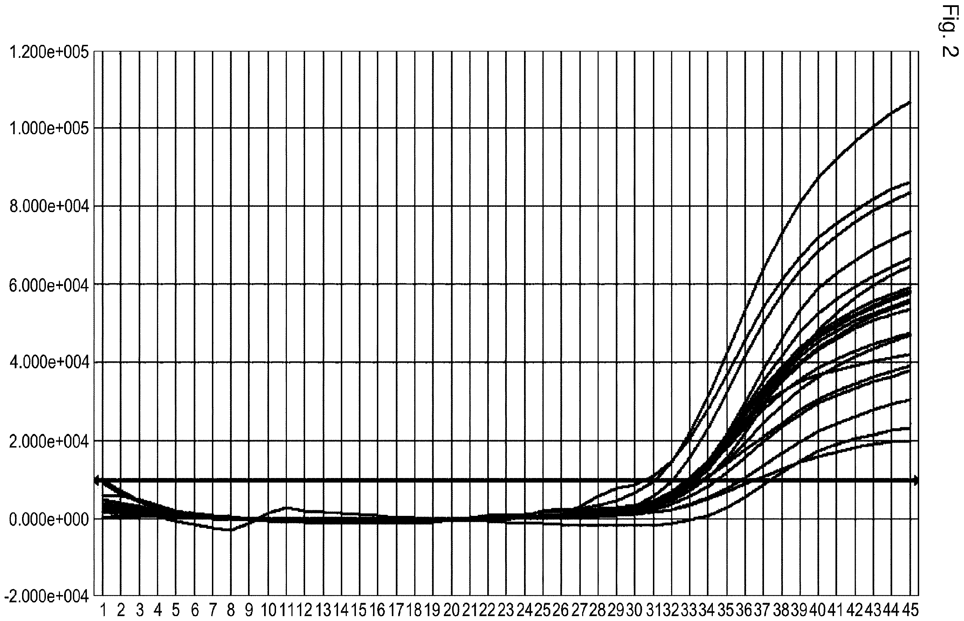

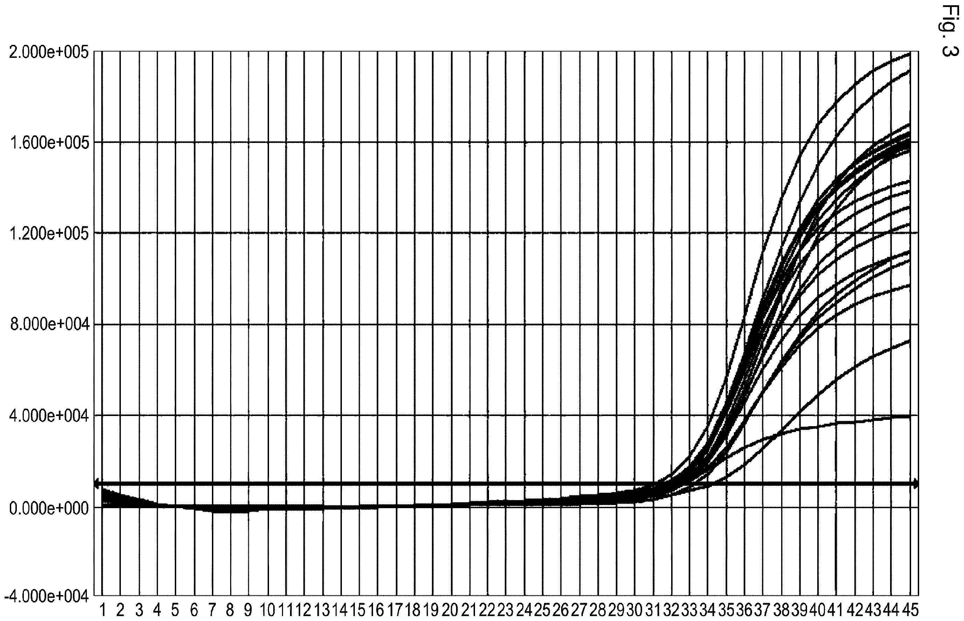

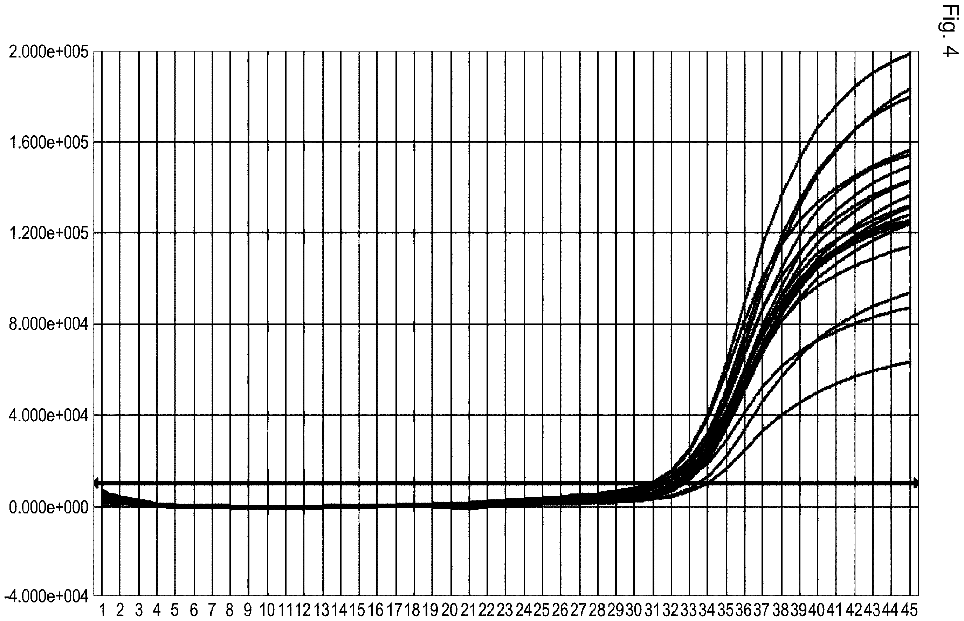

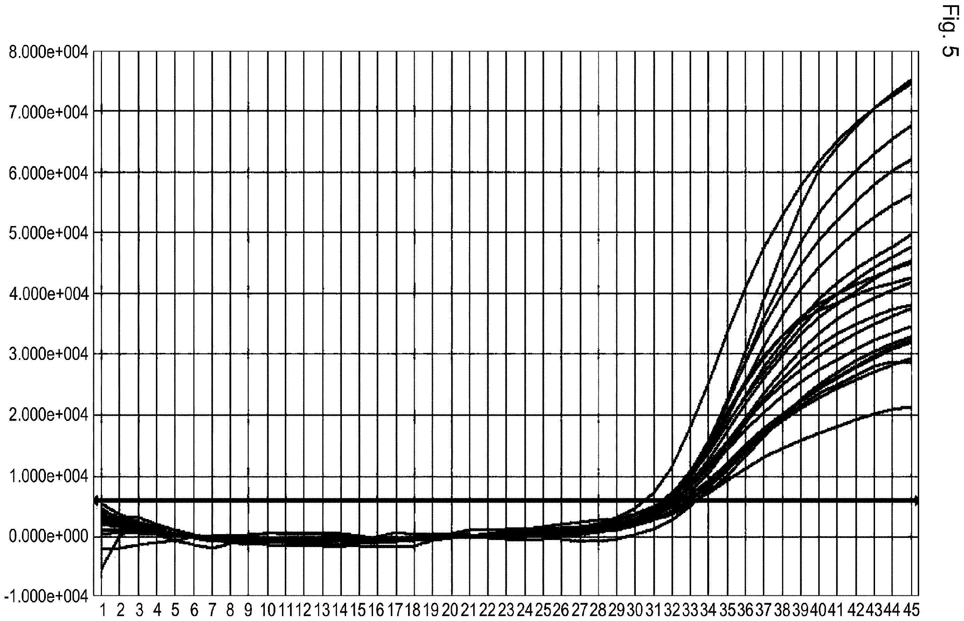

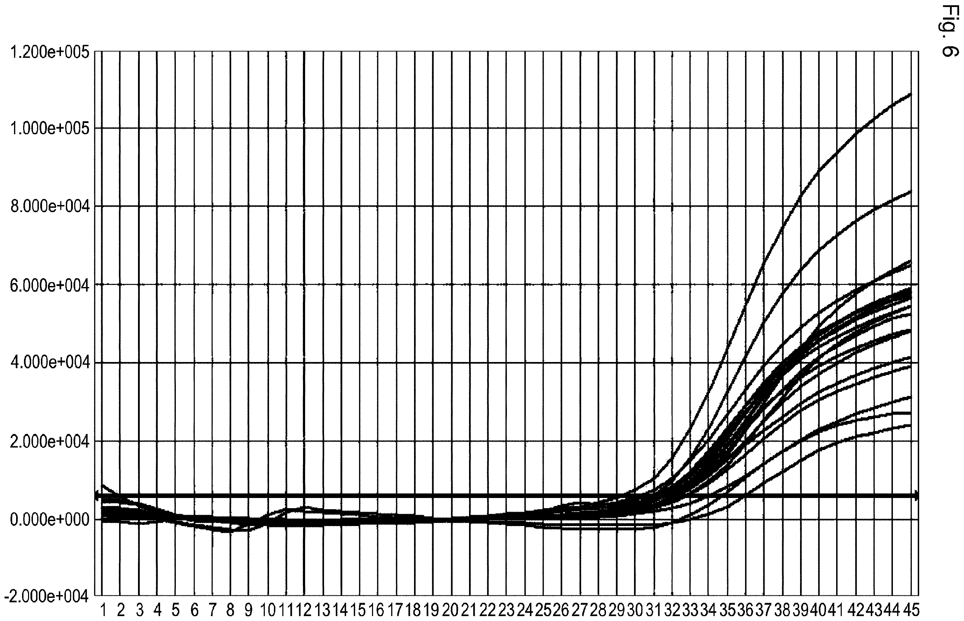

Using the composition in Table 1 of the present invention, according to the method described in Example 2, each target sensitivity positive sample was subjected to detection gradient dilution, and the sample diluted to a detection limit was used as a test sample for detection, and the detection was carried out in each channel 20 times. The composition of the present invention had a detection sensitivity of 400 copies/mL for detection of a 2019 novel coronavirus, a detection sensitivity of 6.5 TCID₅₀/mL for detection of an influenza A virus, a detection sensitivity of 3.0 TCID₅₀/mL for detection of an influenza B virus, a detection sensitivity of 500 copies/mL for detection of a respiratory syncytial virus, a detection sensitivity of 400 copies/mL for detection of an adenovirus, and a detection sensitivity of 400 copies/mL for detection of *Mycoplasma pneumoniae.* The experimental results are as shown in FIGs. 1-6.

### Example 5. Specificity of the composition of the present invention

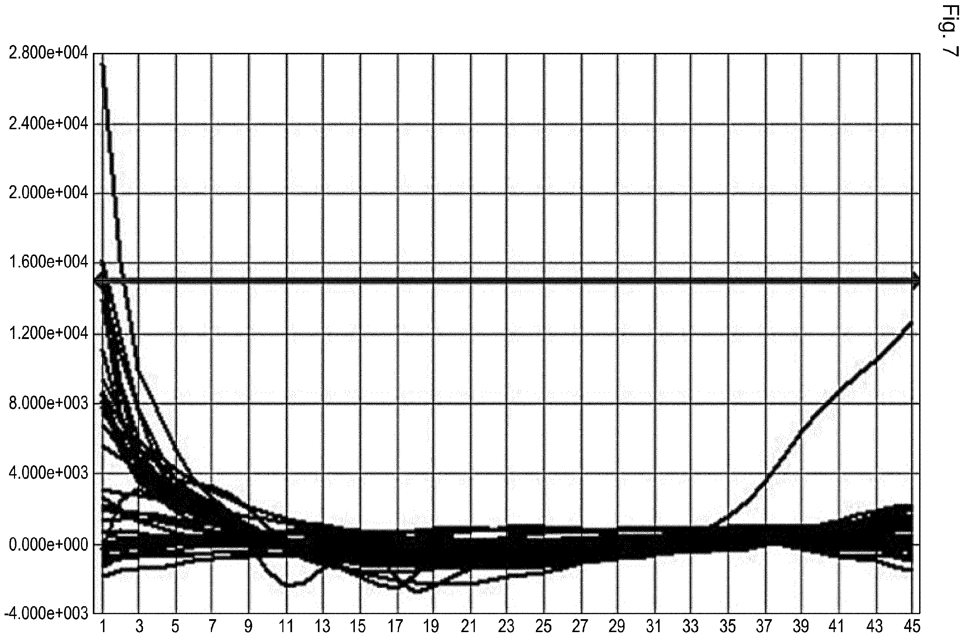

The composition of the present invention was used to detect other common respiratory tract pathogens. Experiments showed that the composition of the present invention had no cross-reaction with common respiratory tract pathogens (measles virus, mumps virus, rubella virus, *Staphylococcus aureus, Escherichia coli, Pseudomonas aeruginosa,* human parainfluenza virus type I, cytomegalovirus, Coxsackie virus type A, human metapneumovirus, *Bacillus pertussis, Chlamydia pneumoniae, Haemophilus influenzae, Streptococcus salivarius, Streptococcus pneumoniae, Neisseria meningitidis, Mycobacterium tuberculosis,* etc.). The experimental results are as shown in FIG. 7.

### Example 6. Effects of interfering substances on the composition of the present invention

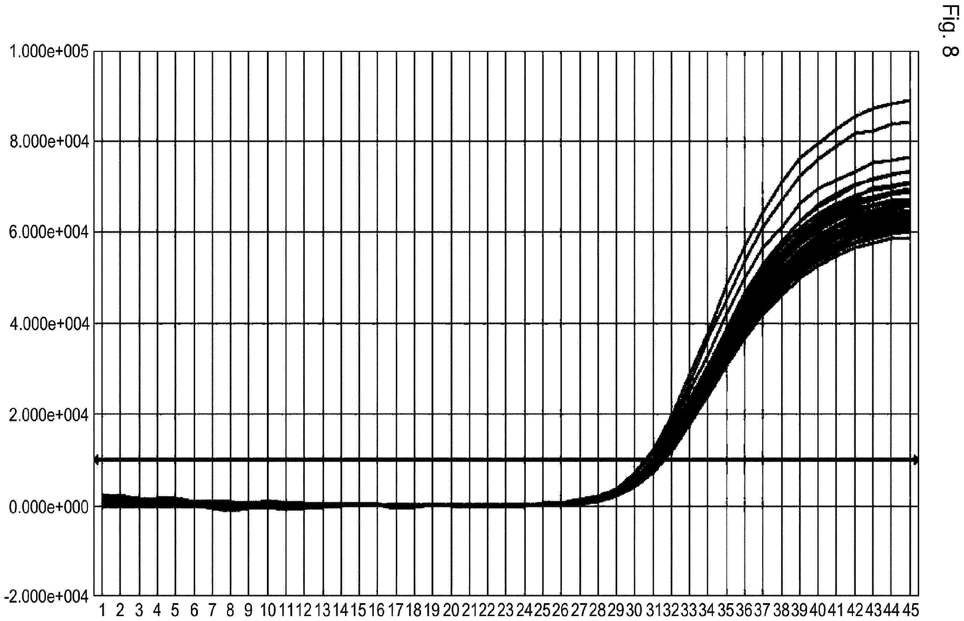

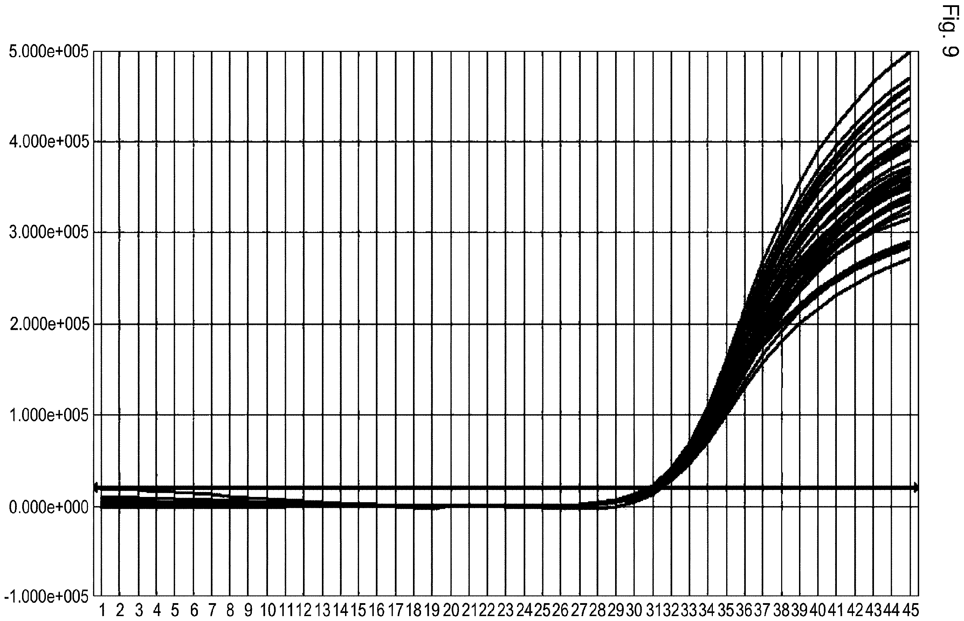

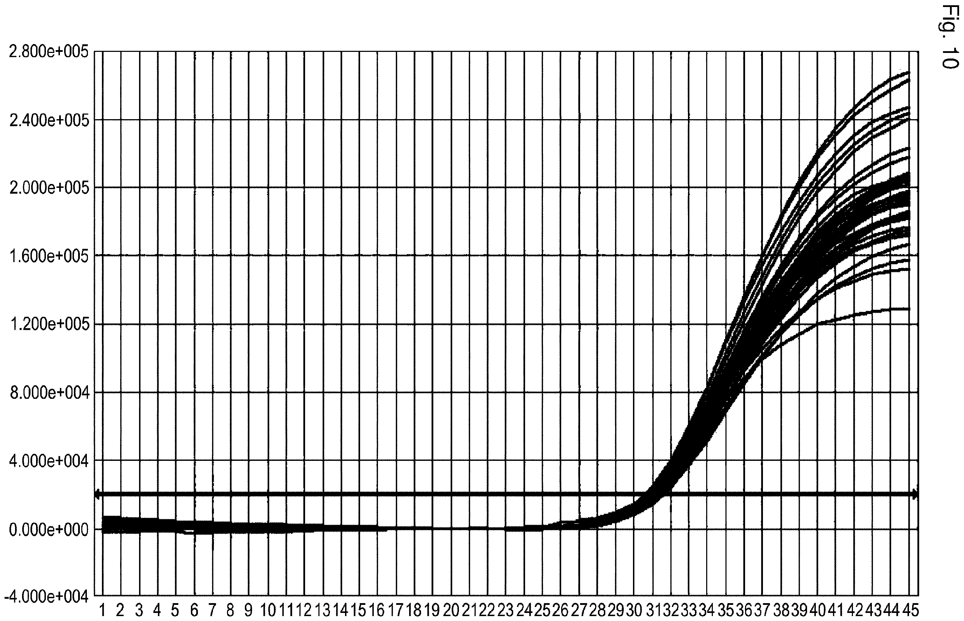

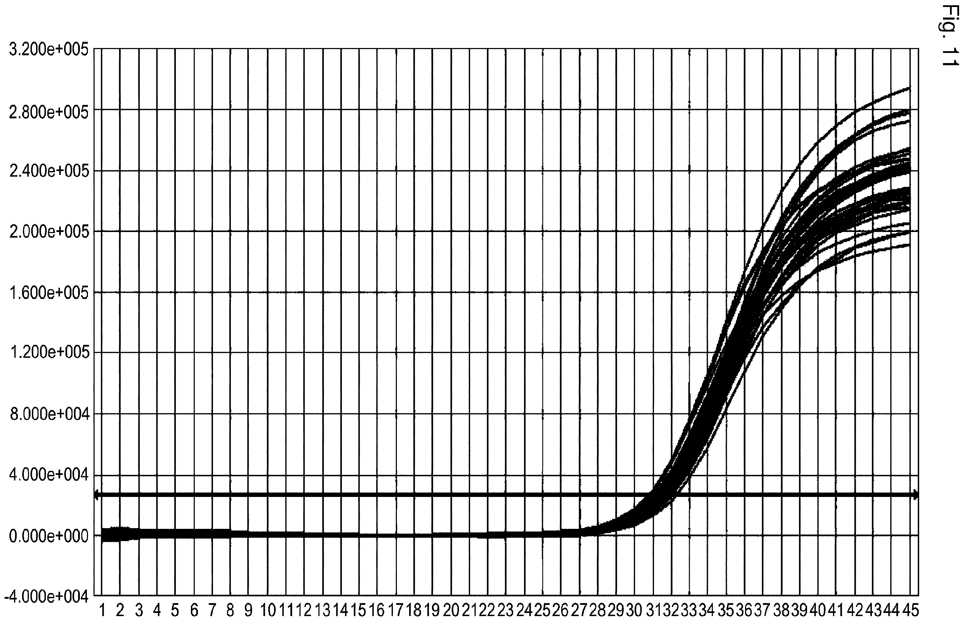

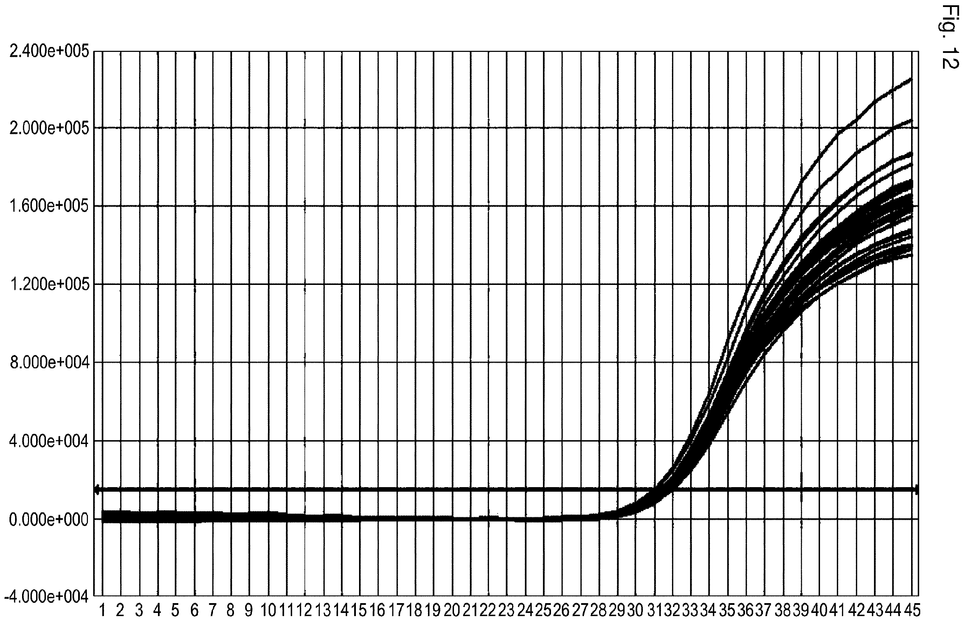

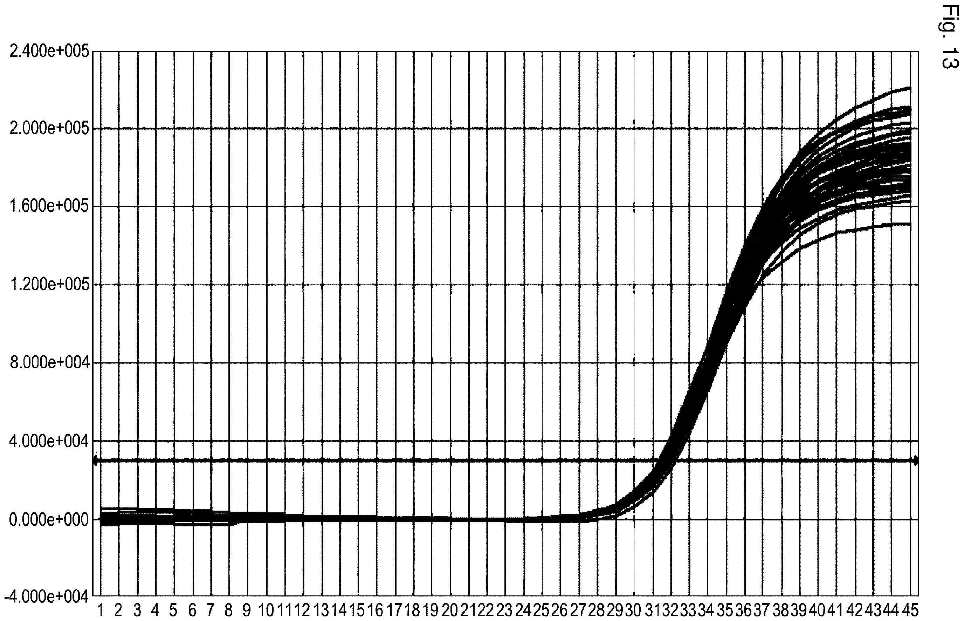

The detection with this kit was not affected by the addition of a certain proportion of a therapeutic drug such as a common cold medicine, a glucocorticoid, an antibiotic, etc. respectively into the samples. Samples containing oxymetazoline hydrochloride (100 µg/mL), dexamethasone (50 µg/mL), cefmenoxime hydrochloride (50 µg/mL), menthol (50 µg/mL), zanamivir (100 µg/mL), ribavirin (100 µg/mL), and azithromycin (100 µg/mL) were tested respectively. The results showed that these interferences had no effect on the detection results and did not affect the accuracy of the composition of the present invention. The experimental results are as shown in FIGs. 8-13.

### Example 7. Joint detection of multiple pathogens, and mutual interference between primers and probes

Due to the principle of complementary base pairing, dimers will be formed between primers and/or probes, but this probability is low and can be ruled out at the beginning of design. However, when multiple pathogens are jointly detected, there are numerous primers and probes, and dimers are prone to form between a primer and a primer, a probe and a probe, or a primer and a probe. In order to ensure the conservation of the design (conservation is critical to the accuracy of detection) and take mutual interference between different primers and probes into consideration, the primers and probes need to be carefully designed.

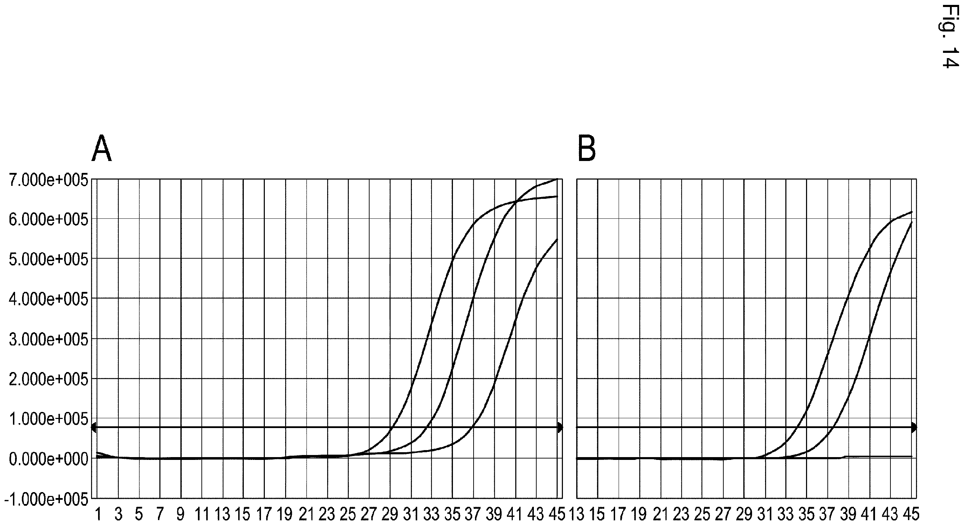

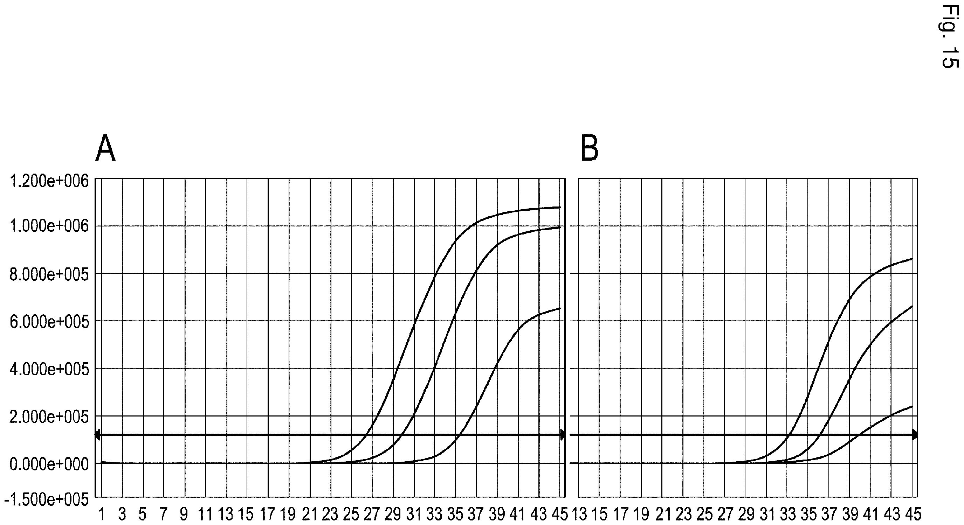

In the present invention, the inventor replaced the *Mycoplasma pneumoniae* in the present invention with a rhinovirus or a metapneumovirus, but through tests, it was found that the addition of the rhinovirus and the metapneumovirus could seriously affect the detection sensitivity of the composition and method of the present invention (the experimental results are shown in FIG. 14 and FIG. 15). It can be seen from the figures that after using the rhinovirus or the metapneumovirus instead, the Ct value shifted backwards significantly, which would affect the sensitivity of the composition and method of the present invention.

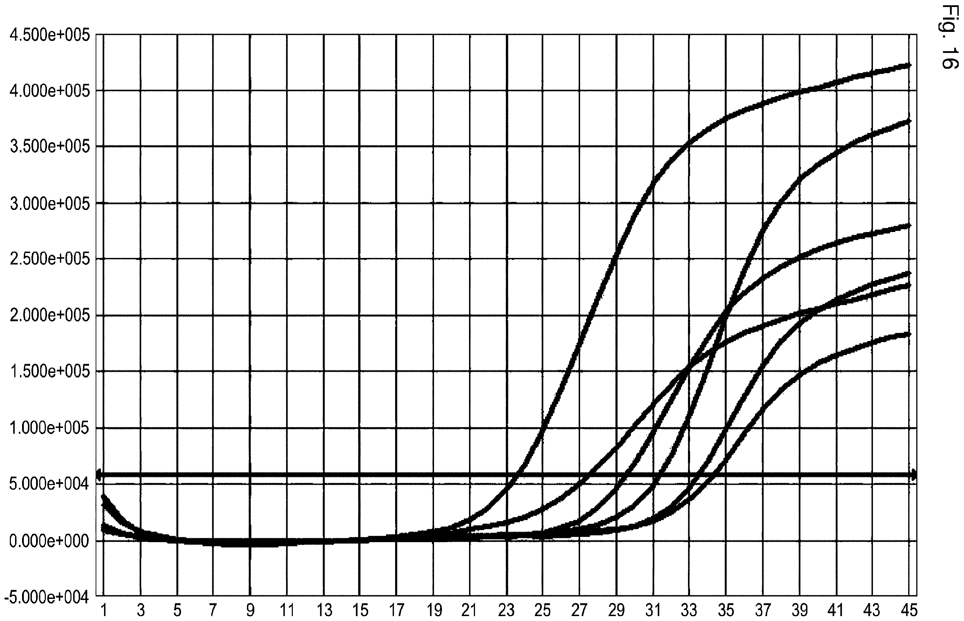

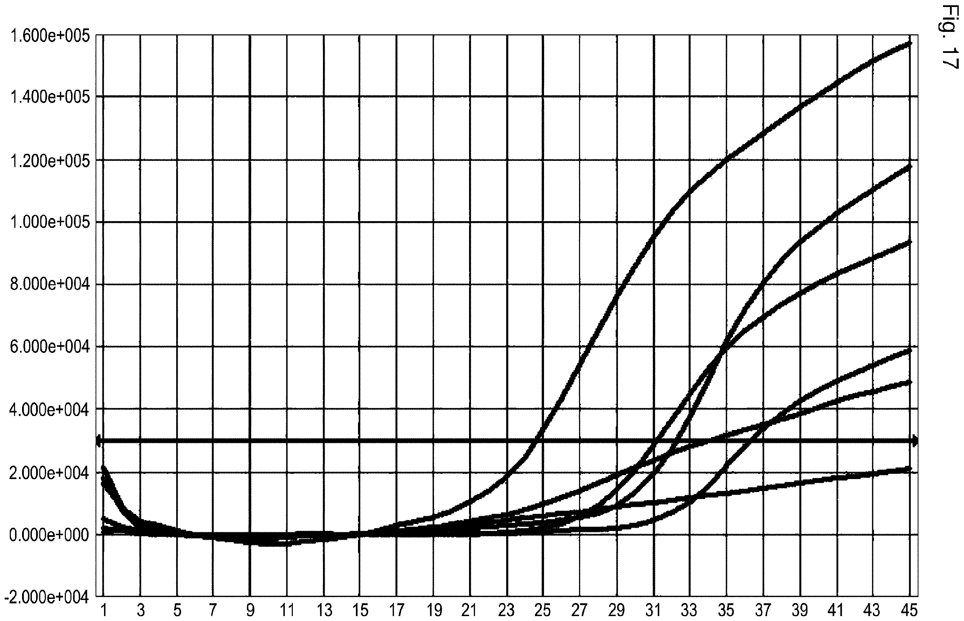

In the present invention, the inventor compared preferred primers and probe for detecting an influenza A virus in the present invention with other primers and probe for detecting an influenza A virus designed by the inventor (the primer sequences were SEQ ID NO: 23: GCGGATGCCTTTTGTTGATT and SEQ ID NO: 24: TTGCTTCAAATGAGAATGTGG; the probe sequence was SEQ ID NO: 25: CCACTCCTGGTCCTTATGGCCCAGT), and simultaneously tested six influenza A virus samples. The experimental results are as shown in FIG. 16 and FIG. 17. The results showed that the other primer and probes not preferred in the present invention seriously affected the detection results of the entire system.

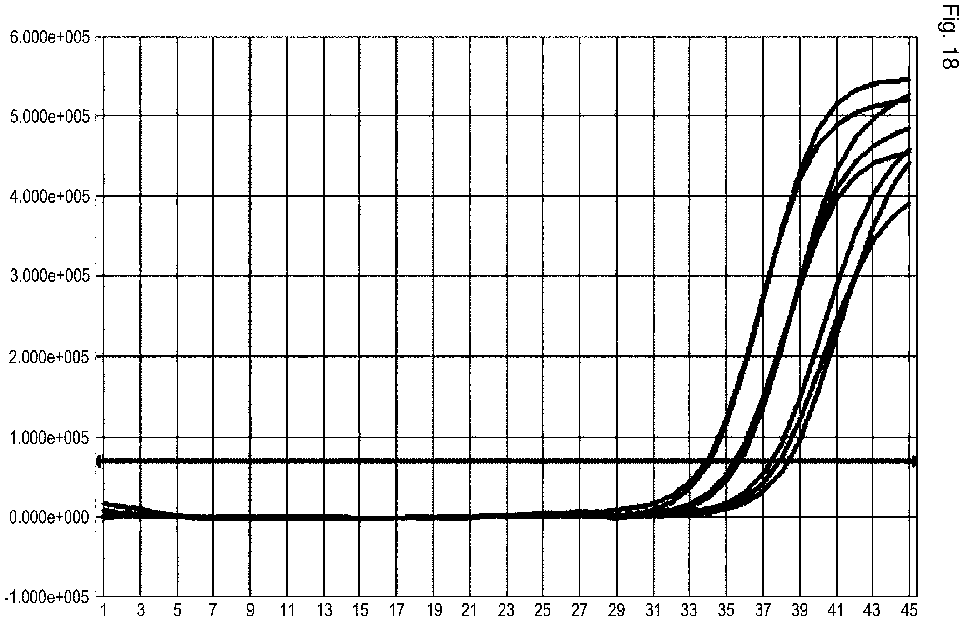

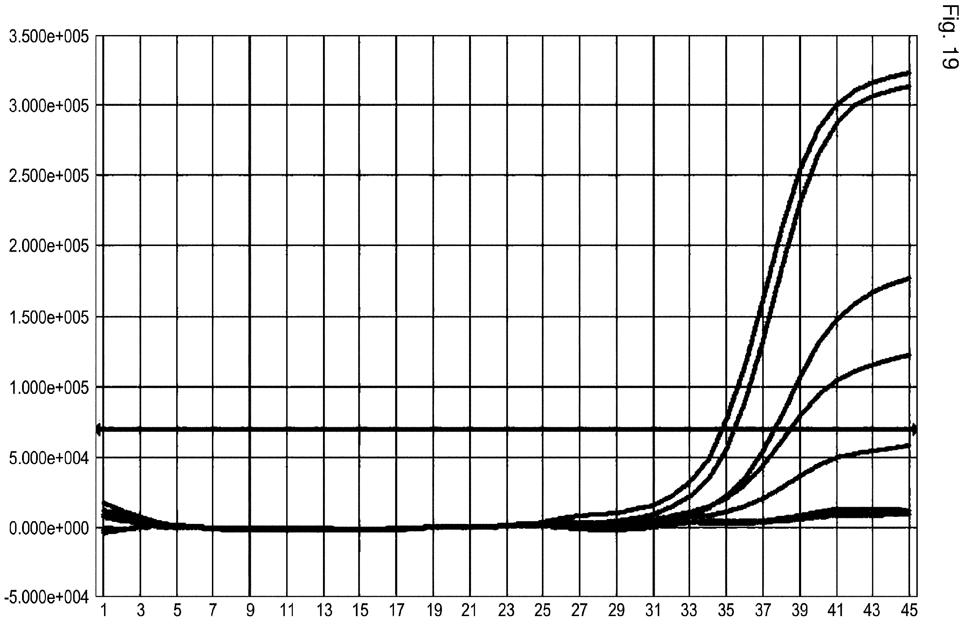

In the present invention, the inventor compared preferred primers and probe for detecting a novel coronavirus 2019-nCoV in the present invention with other primers and probe designed by the inventor for detecting a novel coronavirus 2019-nCoV (the primer sequences were SEQ ID NO: 26: CACTGATGACAATGCGTTAGCTTAC and SEQ ID NO: 27: CCAGTTCTGTATAGATAGTACCAGTTCCA; the probe sequence was SEQ ID NO: 28: CGGATAACAGTGCAAGTACAAACCTACCT). The concentration of a novel coronavirus 2019-nCoV standard was diluted into three gradients (2000 copies/mL, 500 copies/mL, and 200 copies/mL). Experimental results are as shown in FIG. 18 and FIG. 19. The results showed that the other primers and probes not preferred in the present invention seriously affected the detection results of the entire system.

Therefore, the composition of the present invention is unique.

## Claims

1. A composition capable of detecting and typing pathogens that cause a respiratory tract infection, the composition comprising:
a first nucleic acid composition:
an influenza A virus forward primer as shown in SEQ ID NO: 1, an influenza A virus reverse primer as shown in SEQ ID NO: 2, and an influenza A virus probe as shown in SEQ ID NO: 3;
an influenza B virus forward primer as shown in SEQ ID NO: 4, an influenza B virus reverse primer as shown in SEQ ID NO: 5, and an influenza B virus probe as shown in SEQ ID NO: 6;
a respiratory syncytial virus forward primer as shown in SEQ ID NO: 7, a respiratory syncytial virus reverse primer as shown in SEQ ID NO: 8, and a respiratory syncytial virus probe as shown in SEQ ID NO: 9; and
a second nucleic acid composition:
a respiratory adenovirus forward primer as shown in SEQ ID NO: 10, a respiratory adenovirus reverse primer as shown in SEQ ID NO: 11, and a respiratory adenovirus probe as shown in SEQ ID NO: 12;
a novel coronavirus 2019-nCoV forward primer as shown in SEQ ID NO: 13, a novel coronavirus 2019-nCoV reverse primer as shown in SEQ ID NO: 14, and a novel coronavirus 2019-nCoV probe as shown in SEQ ID NO: 15;
*a Mycoplasma pneumoniae* forward primer as shown in SEQ ID NO: 16, a *Mycoplasma pneumoniae* reverse primer as shown in SEQ ID NO: 17, and a *Mycoplasma pneumoniae* probe as shown in SEQ ID NO: 18;
wherein fluorescent groups of the first nucleic acid composition are different from each other and do not interfere with each other, and fluorescent groups of the second nucleic acid composition are different from each other and do not interfere with each other.

2. The composition according to claim 1, wherein the composition further comprises an internal standard forward primer, internal standard reverse primer, and internal standard probe for monitoring.

3. The composition according to claim 2, wherein the internal standard forward primer is as shown in SEQ ID NO: 19, the internal standard reverse primer is as shown in SEQ ID NO: 20, and the internal standard probe is as shown in SEQ ID NO: 21.

4. The composition according to claim 1, wherein the fluorescent reporter group of the influenza A virus probe as shown in SEQ ID NO: 3 is FAM; the fluorescent reporter group of the influenza B virus probe as shown in SEQ ID NO: 6 is HEX; the fluorescent reporter group of the respiratory syncytial virus probe as shown in SEQ ID NO: 9 is CY5; the fluorescent reporter group of the respiratory adenovirus probe as shown in SEQ ID NO: 12 is FAM; the fluorescent reporter group of the novel coronavirus 2019-nCoV probe as shown in SEQ ID NO: 15 is HEX; and the fluorescent reporter group of the *Mycoplasma pneumoniae* probe as shown in SEQ ID NO: 18 is CY5.

5. The composition according to any one of claims 1 to 4, wherein the two nucleic acid compositions are each present in separate packages.

6. A use of the composition according to any one of claims 1 to 5 in the preparation of a kit for detecting and typing pathogens that cause a respiratory tract infection.

7. A kit for detecting and typing pathogens that cause a respiratory tract infection, the kit comprising the composition according to any one of claims 1 to 5.

8. The kit according to claim 7, wherein the kit further comprises at least one of a nucleic acid release reagent, a dNTP, a reverse transcriptase, a DNA polymerase, a PCR buffer, and Mg2+.

9. The kit according to claim 8, wherein the amount of the detection primer in the composition is 25-500 nM; the amount of the detection probe in the composition is 20-500 nM; the amount of the internal standard primer in the composition is 12.5-250 nM; and the amount of the internal standard probe in the composition is 10-250 nM.

10. A method for detecting and typing pathogens that cause a respiratory tract infection, the method comprising the steps of:
1) releasing a nucleic acid of a testing sample;
2) performing, by using the composition according to any one of claims 1 to 5, a fluorescent quantitative PCR on the nucleic acid obtained in step 1); and
3) obtaining and analyzing the results.

## Patentansprüche

1. Zusammensetzung, die in der Lage ist, Krankheitserreger, die eine Atemwegsinfektion verursachen, nachzuweisen und zu typisieren, wobei die Zusammensetzung umfasst:
eine erste Nukleinsäurezusammensetzung:
einen Influenza-A-Virus-Vorwärtsprimer wie in SEQ ID NO: 1 gezeigt, einen Influenza-A-Virus-Rückwärtsprimer wie in SEQ ID NO: 2 gezeigt und eine Influenza-A-Virus-Sonde wie in SEQ ID NO: 3 gezeigt;
einen Influenza-B-Virus-Vorwärtsprimer wie in SEQ ID NO: 4 gezeigt, einen Influenza-B-Virus-Rückwärtsprimer wie in SEQ ID NO: 5 gezeigt und eine Influenza-B-Virus-Sonde wie in SEQ ID NO: 6 gezeigt;
einen Respiratory-Syncytial-Virus-Vorwärtsprimer wie in SEQ ID NO: 7 gezeigt, einen Respiratory-Syncytial-Virus-Rückwärtsprimer wie in SEQ ID NO: 8 gezeigt und eine Respiratory-Syncytial-Virus-Sonde wie in SEQ ID NO: 9 gezeigt; und
eine zweite Nukleinsäurezusammensetzung:
einen respiratorischen Adenovirus-Vorwärtsprimer wie in SEQ ID NO: 10 gezeigt, einen respiratorischen Adenovirus-Rückwärtsprimer wie in SEQ ID NO: 11 gezeigt und eine respiratorische Adenovirus-Sonde wie in SEQ ID NO: 12 gezeigt;
einen neuartigen Coronavirus 2019-nCoV-Vorwärtsprimer wie in SEQ ID NO: 13 gezeigt, einen neuartigen Coronavirus 2019-nCoV-Rückwärtsprimer wie in SEQ ID NO: 14 gezeigt und eine neuartige Coronavirus 2019-nCoV-Sonde wie in SEQ ID NO: 15 gezeigt;
einen *Mycoplasma* pneumoniae-Vorwärtsprimer wie in SEQ ID NO: 16 gezeigt, einen *Mycoplasma* pneumoniae-Rückwärtsprimer wie in SEQ ID NO: 17 gezeigt und eine *Mycoplasma* pneumoniae-Sonde wie in SEQ ID NO: 18 gezeigt;
wobei die fluoreszierenden Gruppen der ersten Nukleinsäurezusammensetzung voneinander verschieden sind und sich nicht gegenseitig stören, und die fluoreszierenden Gruppen der zweiten Nukleinsäurezusammensetzung voneinander verschieden sind und sich nicht gegenseitig stören.

2. Zusammensetzung nach Anspruch 1, wobei die Zusammensetzung ferner einen internen Standard-Vorwärtsprimer, einen internen Standard-Rückwärtsprimer und eine interne Standardsonde zur Überwachung umfasst.

3. Zusammensetzung nach Anspruch 2, wobei der interne Standard-Vorwärtsprimer wie in SEQ ID NO: 19 gezeigt ist, der interne Standard-Rückwärtsprimer wie in SEQ ID NO: 20 gezeigt ist und die interne Standardsonde wie in SEQ ID NO: 21 gezeigt ist.

4. Zusammensetzung nach Anspruch 1, wobei die fluoreszierende Reportergruppe der Influenza-A-Virus-Sonde, wie in SEQ ID NO: 3 gezeigt, FAM ist; die fluoreszierende Reportergruppe der Influenza-B-Virus-Sonde, wie in SEQ ID NO: 6 gezeigt, HEX ist; die fluoreszierende Reportergruppe der Respiratory-Syncytial-Virus-Sonde, wie in SEQ ID NO: 9 ist CYS; die fluoreszierende Reportergruppe der respiratorischen Adenovirus-Sonde wie in SEQ ID NO: 12 gezeigt ist FAM; die fluoreszierende Reportergruppe der neuartigen Coronavirus 2019-nCoV-Sonde wie in SEQ ID NO: 15 gezeigt ist HEX; und die fluoreszierende Reportergruppe der Mycoplasma pneumoniae-Sonde wie in SEQ ID NO: 18 gezeigt ist CYS.

5. Zusammensetzung nach einem der Ansprüche 1 bis 4, wobei die beiden Nukleinsäurezusammensetzungen jeweils in getrennten Verpackungen vorliegen.

6. Verwendung der Zusammensetzung nach einem der Ansprüche 1 bis 5 zur Herstellung eines Kits zum Nachweis und zur Typisierung von Pathogenen, die eine Atemwegsinfektion verursachen.

7. Kit zum Nachweis und zur Typisierung von Erregern, die eine Atemwegsinfektion verursachen, wobei das Kit die Zusammensetzung nach einem der Ansprüche 1 bis 5 enthält.

8. Kit nach Anspruch 7, wobei das Kit ferner mindestens eines der folgenden Elemente umfasst: ein Nukleinsäure-Freisetzungsreagenz, ein dNTP, eine reverse Transkriptase, eine DNA-Polymerase, einen PCR-Puffer und Mg2+.

9. Kit nach Anspruch 8, wobei die Menge des Detektionsprimers in der Zusammensetzung 25-500 nM beträgt; die Menge der Detektionssonde in der Zusammensetzung 20-500 nM beträgt; die Menge des internen Standardprimers in der Zusammensetzung 12,5-250 nM beträgt; und die Menge der internen Standardsonde in der Zusammensetzung 10-250 nM beträgt.

10. Verfahren zum Nachweis und zur Typisierung von Krankheitserregern, die eine Atemwegsinfektion verursachen, wobei das Verfahren die folgenden Schritte umfasst:
1) Freisetzung einer Nukleinsäure aus einer Testprobe;
2) Durchführen einer fluoreszierenden quantitativen PCR an der in Schritt 1) erhaltenen Nukleinsäure unter Verwendung der Zusammensetzung nach einem der Ansprüche 1 bis 5; und
3) Gewinnung und Analyse der Ergebnisse.

## Revendications

1. Une composition capable de détecter et de typer les pathogènes responsables d'une infection des voies respiratoires, la composition comprenant :
une première composition d'acides nucléiques :
une amorce sens du virus de la grippe A comme présentée dans SEQ ID NO : 1, une amorce anti-sens du virus de la grippe A comme présenté dans SEQ ID NO : 2, et une sonde du virus de la grippe A comme présenté dans SEQ ID NO : 3 ;
une amorce sens du virus de la grippe B comme présenté dans SEQ ID NO : 4, une amorce anti-sens du virus de la grippe B comme présenté dans SEQ ID NO : 5, et une sonde du virus de la grippe B comme présenté dans SEQ ID NO : 6 ;
une amorce sens du virus respiratoire syncytial telle que présentée dans SEQ ID NO : 7, une amorce anti-sens du virus respiratoire syncytial comme présentée dans SEQ ID NO : 8, et une sonde du virus respiratoire syncytial comme présentée dans SEQ ID NO : 9 ; et
une deuxième composition d'acides nucléiques :
une amorce sens d'adénovirus respiratoire comme présentée dans SEQ ID NO : 10, une amorce anti-sens d'adénovirus respiratoire comme présentée dans SEQ ID NO : 11, et une sonde d'adénovirus respiratoire comme présentée dans SEQ ID NO : 12 ;
une amorce sens du nouveau coronavirus 2019-nCoV comme présentée dans SEQ ID NO : 13, une amorce anti-sens du nouveau coronavirus 2019-nCoV comme présentée dans SEQ ID NO : 14, et une sonde du nouveau coronavirus 2019-nCoV comme présentée dans SEQ ID NO : 15 ;
une amorce sens de *Mycoplasma pneumoniae* comme présentée dans SEQ ID NO : 16, une amorce anti-sens de *Mycoplasma pneumoniae* comme présentée dans SEQ ID NO: 17, et une sonde de *Mycoplasma pneumoniae* comme présentée dans SEQ ID NO : 18 ;
où les groupes fluorescents de la première composition d'acides nucléiques sont différents les uns des autres et n'interfèrent pas entre eux, et les groupes fluorescents de la seconde composition d'acides nucléiques sont différents les uns des autres et n'interfèrent pas entre eux.

2. La composition selon la revendication 1, où la composition comprend en outre une amorce sens standard interne, une amorce anti-sens standard interne, et une sonde standard interne pour le contrôle.

3. La composition selon la revendication 2, où l'amorce sens standard interne est comme présentée dans SEQ ID NO : 19, l'amorce anti-sens standard interne est comme présentée dans SEQ ID NO : 20, et la sonde standard interne comme présentée dans SEQ ID NO : 21.

4. La composition selon la revendication 1, où le groupe rapporteur fluorescent de la sonde du virus de la grippe A comme présentée dans SEQ ID NO : 3 est FAM ; le groupe rapporteur fluorescent de la sonde du virus de la grippe B comme présentée dans SEQ ID NO : 6 est HEX ; le groupe rapporteur fluorescent de la sonde du virus respiratoire syncytial comme présenté dans SEQ ID NO : 9 est CYS ; le groupe rapporteur fluorescent de la sonde de l'adénovirus respiratoire comme présenté dans SEQ ID NO : 12 est FAM ; le groupe rapporteur fluorescent de la sonde du nouveau coronavirus 2019-nCoV comme présenté dans SEQ ID NO : 15 est HEX ; et le groupe rapporteur fluorescent de la sonde de *Mycoplasma pneumoniae* comme présenté dans SEQ ID NO : 18 est CYS.

5. La composition selon l'une quelconque des revendications 1 à 4, où les deux compositions d'acides nucléiques sont présentées dans des emballages distincts.

6. Une utilisation de la composition selon l'une quelconque des revendications 1 à 5 dans la préparation d'un kit de détection et de typage des pathogènes responsables d'une infection des voies respiratoires.

7. Un kit de détection et de typage de pathogènes responsables d'une infection des voies respiratoires, le kit comprenant la composition selon l'une quelconque des revendications 1 à 5.

8. Le kit selon la revendication 7, où le kit comprend en outre au moins un réactif de libération d'acide nucléique, un dNTP, une transcriptase inverse, une ADN polymérase, un tampon PCR et Mg²⁺

9. Le kit selon la revendication 8, où la quantité d'amorce de détection dans la composition est de 25-500 nM ; la quantité de sonde de détection dans la composition est de 20-500 nM ; la quantité d'amorce standard interne dans la composition est de 12,5-250 nM ; et la quantité de sonde standard interne dans la composition est de 10-250 nM.

10. Un procédé de détection et de typage de pathogènes responsables d'une infection des voies respiratoires, comprenant les étapes de :
1) libération d'un acide nucléique d'un échantillon à tester ;
2) réalisation, en utilisant la composition selon l'une quelconque des revendications 1 à 5 ; une PCR quantitative fluorescente sur l'acide nucléique obtenu à l'étape 1) ; et
3) obtention et analyse des résultats.
